(19) Europäisches Patentamt
European Patent Office
Office européen des brevets

(11) Numéro de publication : **0 110 769**
**B1**

(12) # FASCICULE DE BREVET EUROPÉEN

(45) Date de publication du fascicule du brevet : **05.03.86**

(21) Numéro de dépôt : **83402237.8**

(22) Date de dépôt : **21.11.83**

(51) Int. Cl.⁴ : **C 07 C 69/743**, C 07 C153/11, C 07 D207/32, C 07 D401/04, A 01 N 53/00, A 23 L 1/16

(54) **Nouveaux esters dérivés d'acides 2,2-diméthyl cyclopropane carboxyliques et d'alcools biaryliques, leur procédé de préparation, leur application à la lutte contre les parasites et les compositions les renfermant.**

(30) Priorité : **25.11.82 FR 8219761**

(43) Date de publication de la demande : **13.06.84 Bulletin 84/24**

(45) Mention de la délivrance du brevet : **05.03.86 Bulletin 86/10**

(84) Etats contractants désignés : **AT BE CH DE FR GB IT LI LU NL SE**

(56) Documents cités :
**EP-A- 0 048 186**
**EP-A- 0 049 977**
**EP-A- 0 050 534**
**WO-A-82 /013 68**
**FR-A- 2 138 158**
**Le dossier contient des informations techniques présentées postérieurement au dépôt de la demande et ne figurant pas dans le présent fascicule.**

(73) Titulaire : **ROUSSEL-UCLAF**
**35, boulevard des Invalides**
**F-75007 Paris (FR)**

(72) Inventeur : **Tessier, Jean**
**30, rue Jean Moulin**
**F-94300 Vincennes (FR)**
Inventeur : **Demoute, Jean-Pierre**
**249bis, rue de Rosny**
**F-93100 Montreuil-sous-Bois (FR)**

(74) Mandataire : **Niel, Michel et al**
**ROUSSEL-UCLAF Boîte postale no 9 111, route de Noisy**
**F-93230 Romainville (FR)**

**EP 0 110 769 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux esters dérivés d'acides 2,2-diméthyl cyclopropane carboxyliques et d'alcools biaryliques, leur procédé de préparation, leur application à la lutte contre les parasites et les compositions les renfermant.

L'invention a pour objet sous toutes les formes d'isomères possibles ou sous forme de mélanges d'isomères, les composés de formule (I) :

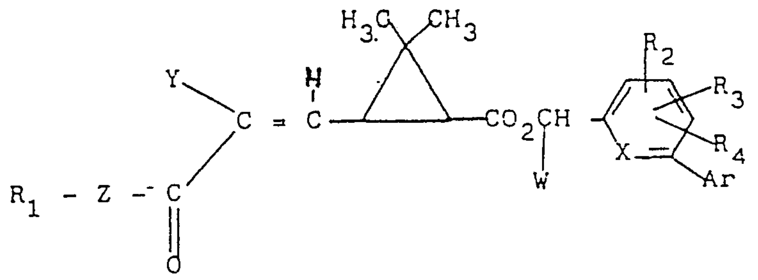

(I)

dans laquelle Y représente un atome d'hydrogène ou d'halogène ou un radical —Oalc ou

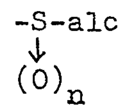

dans lequel alc est un radical alcoyle renfermant de 1 à 6 atomes de carbone et n est égal à 0, 1 ou 2,

Z représente un atome d'oxygène ou de soufre, $R_1$ représente

ou bien un radical alcoyle, linéaire, ramifié ou cyclique, saturé ou insaturé, renfermant de 1 à 8 atomes de carbone, éventuellement substitué par un ou plusieurs groupements fonctionnels, identiques ou différents,

ou bien un groupement aryle renfermant de 6 à 14 atomes de carbone, éventuellement substitué par un ou plusieurs groupements fonctionnels, identiques ou différents,

ou bien un radical hétérocyclique éventuellement substitué par un ou plusieurs groupements fonctionnels, identiques ou différents, $R_2$, $R_3$ et $R_4$, identiques ou différents, représentent un atome d'hydrogène, un atome d'halogène, un radical alcoyle renfermant jusqu'à 8 atomes de carbone, un radical haloalcoyle renfermant jusqu'à 8 atomes de carbone pouvant renfermer plusieurs atomes d'halogène, un radical alcoxy renfermant jusqu'à 8 atomes de carbone, W représente un atome d'hydrogène ou un radical cyano, X représente un atome de carbone ou un atome d'azote, Ar représente un radical phényle, pyridyle, thiényle, naphtyle, furyle ou pyrrolyle éventuellement substitué et la double liaison éthylénique à la géométrie E ou Z.

Les composés de formule I peuvent exister sous de nombreuses formes stéréoisomères, ils possèdent, en effet, deux carbones assymétriques en 1 et en 3 du cyclopropane ; ils présentent aussi une possibilité d'isomérie E/Z au niveau de la double liaison ; ils peuvent présenter également une ou plusieurs possibilités d'isomérie dans la partie alcoolique.

Lorsque Y représente un atome d'halogène, il s'agit de préférence d'un atome de fluor, de chlore ou de brome.

Alc peut représenter un radical méthyle, éthyle, propyle linéaire ou ramifié ou butyle linéaire ou ramifié.

Lorsque $R_1$ représente un radical alcoyle saturé, linéaire ou ramifié, il s'agit de préférence d'un radical méthyle, éthyle, n-propyle, isopropyle, n-butyle, sec-butyle, isobutyle, n-pentyle, n-hexyle, tert-butyle, tert-pentyle ou néopentyle.

Lorsque $R_1$ représente un radical cyclique, il s'agit de préférence d'un radical cyclopropyle, cyclobutyle, cyclopentyle ou cyclohexyle, d'un radical alcoyle, linéaire ou ramifié, portant un reste cyclique éventuellement substitué par un ou plusieurs radicaux alcoyles par exemple, le radical 1-méthyl cyclobutyle, 1-méthyl cyclopentyle, 1-méthyl cyclohexyle ou 2,2,3,3-tétraméthylcyclopropyle.

Lorsque $R_1$ représente un radical alcoyle insaturé, il s'agit d'un radical éthylénique, comme, par exemple, d'un radical vinyle ou 1,1-diméthylallyle ou d'un radical acétylénique, comme, par exemple, le radical éthynyle ou propynyle.

Lorsque $R_1$ représente un radical alcoyle substitué par un ou plusieurs groupements fonctionnels, on entend par groupement fonctionnel, un atome d'halogène, un groupement OH ou SH, un groupement OR' ou SR' dans lesquels R' représente un radical alcoyle renfermant de 1 à 8 atomes de carbone, un groupement $—NO_2$,

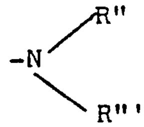

dans lequel R″ et R‴, identiques ou différents, représentent un atome d'hydrogène ou un radical alcoyle renfermant de 1 à 8 atomes de carbone, un groupement —C≡N, $SO_3H$ ou $PO_4H_2$ ou un groupement —COalc$_1$, $SO_2$alc$_2$ ou $SO_3$alc$_3$ dans lesquels alc$_1$, alc$_2$ et alc$_3$ représentent des radicaux alcoyles renfermant de 1 à 18 atomes de carbone.

$R_1$ peut représenter également un radical alcoyle substitué par un radical aryle comme, par exemple, le radical benzyle ou le radical phénéthyle, lui-même éventuellement substitué par un ou plusieurs groupements —OH, Oalc ou alc renfermant de 1 à 8 atomes de carbone, par un ou plusieurs halogènes ou groupements —$CF_3$, $OCF_3$, —$SCF_3$ ou par un groupement (G) :

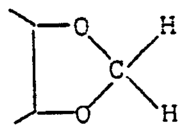

(G)

$R_1$ peut représenter également un radical alcoyle substitué sur deux carbones adjacents par un groupement ($G_1$)

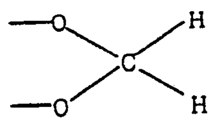

($G_1$)

ou $R_1$ peut être substitué par un groupement

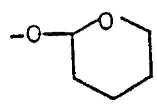

Lorsque $R_1$ représente un radical alcoyle substitué par un ou plusieurs groupements fonctionnels, on peut citer comme valeurs préférées de $R_1$, les radicaux :

—$(CH_2)n_1$ —$CHal_3$ dans lequel $n_1$ est un entier de 1 à 8 et Hal un atome d'halogène, par exemple, le radical —$CH_2$—$CCl_3$, —$CH_2$—$CF_3$, —$CH_2$—$CH_2$—$CCl_3$ ou —$CH_2$—$CH_2$—$CF_3$, —$(CH_2)n_2$ —$CHHal_2$ dans lequel Hal est défini comme ci-dessus et $n_2$ est un nombre de 0 à 8, par exemple, le radical :

—$CH_2$—$CHCl_2$, —$CH_2$—$CHF_2$ ou $CHF_2$,

—$(CH_2)n$, —$CH_2$ Hal dans lequel n′ est un nombre de 0 à 8 et Hal est défini comme ci-dessus, par exemple, le radical :

—$CH_2$—$CH_2Cl$ ou —$CH_2$—$CH_2F$,

—C≡$(CHal_3)_3$ dans lequel Hal est défini comme ci-dessus, par exemple, le radical :

$-C\equiv(CF_3)_3$ . ou 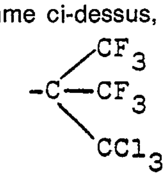

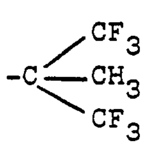 , 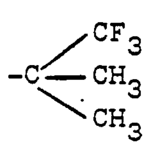 ou 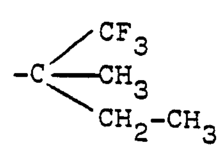

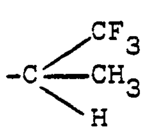 ou 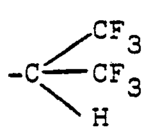

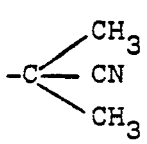 , 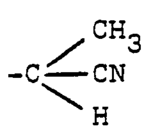 ou —$(CH_2)n_1$—CN,

dans lequel $n_1$ est défini comme précédemment,

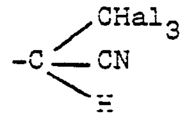

dans lequel Hal est défini comme précédemment, par exemple, le radical :

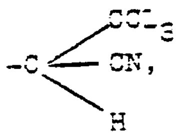

$-(CH_2)n'-OR_5$ dans lequel n' est défini comme précédemment et $R_5$ représente un atome d'hydrogène ou un radical alcoyle, linéaire ou ramifié, comportant de 1 à 8 atomes de carbone, par exemple, le radical : $-CH_2-OCH_3$, $-CH_2-CH_2-O-CH_3$, $-CH_2-CH_2-O-CH_2-CH_3$ ou $-CH_2-CH_2-OH$,

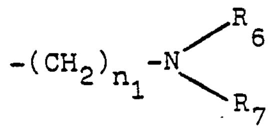

dans lequel $n_1$ est défini comme précédemment, et les deux radicaux $R_6$ et $R_7$ représentent un atome d'hydrogène ou un radical alcoyle renfermant de 1 à 8 atomes de carbone, par exemple le radical :

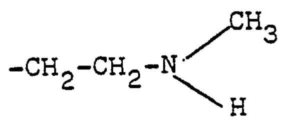

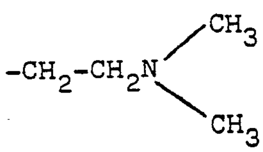 ou 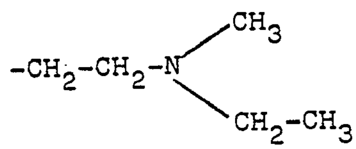 ,

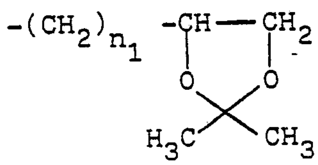 ,

dans lequel $n_1$ est défini comme précédemment, par exemple, le radical :

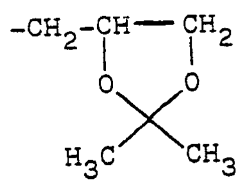 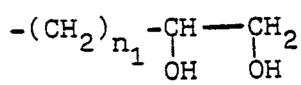

dans lequel $n_1$ est défini comme précédemment, par exemple, le radical :

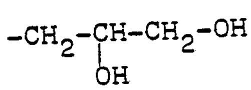 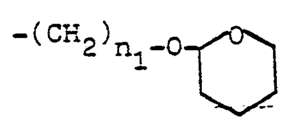

dans lequel $n_1$ est défini comme précédemment, par exemple, le radical :

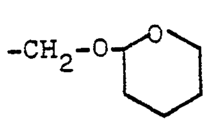 ou 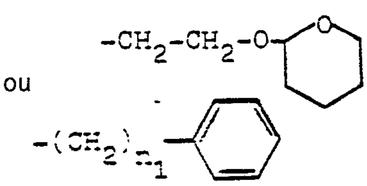

dans lequel $n_1$ est défini comme précédemment, par exemple, le radical benzyle ou phénéthyle,

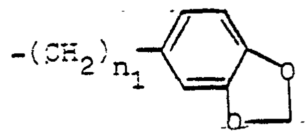

dans lequel $n_1$ est défini comme précédemment, par exemple, le radical :

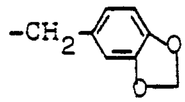

Lorsque $R_1$ représente un radical aryle, éventuellement substitué, il s'agit de préférence du radical phényle ou du radical phényle substitué par un ou plusieurs groupements OH, Oalc et alc, renfermant de 1 à 8 atomes de carbone ou par un halogène ou un groupement $—CF_3$, $—OCF_3$ ou $SCF_3$.

Lorsque $R_1$ représente un radical hétérocyclique, il s'agit de préférence du radical pyridinyle, furanyle, thiophényle, oxazolyle ou thiazolyle.

Lorsque $R_2$, $R_3$ ou $R_4$ représente un atome d'halogène, on entend de préférence, par halogène, un atome de fluor, de chlore ou de brome.

Lorsque $R_2$, $R_3$ ou $R_4$ représente un radical alcoyle, il s'agit de préférence du radical méthyle ou éthyle.

Lorsque $R_2$, $R_3$ ou $R_4$ représente un radical haloalcoyle, il s'agit de préférence du radical $CF_3$ ou $CH_2CF_3$.

Lorsque $R_2$, $R_3$ ou $R_4$ représente un radical alcoxy, il s'agit de préférence du radical méthoxy, éthoxy ou propoxy.

Lorsque Ar représente un radical aryle substitué, il s'agit de préférence d'un radical aryle substitué par un ou plusieurs atomes d'halogène, par un ou plusieurs radicaux haloalcoyles renfermant jusqu'à 8 atomes de carbone, par un ou plusieurs radicaux alcoyles ou alcoxy renfermant jusqu'à 8 atomes de carbone, il s'agit, par exemple, d'un radical aryle substitué par un ou plusieurs atomes de fluor, de chlore ou de brome, par un ou plusieurs radicaux trifluorométhyle, par un ou plusieurs radicaux méthyle ou méthoxy.

Parmi les composés de formule (I), on peut citer ceux dans lesquels Y représente un atome d'hydrogène ou d'halogène, $R_2$, $R_3$ et $R_4$ sont identiques, W représente un atome d'hydrogène et X représente un atome de carbone.

L'invention a plus particulièrement pour objet les composés pour lesquels la structure de la copule cyclopropanique est 1R, cis.

Parmi les composés de l'invention, on peut citer les composés pour lesquels Ar représente un radical pyrrolyle ou un radical phényle, ainsi que ceux pour lesquels un ou plusieurs des radicaux $R_2$, $R_3$ et $R_4$ représentent un radical alcoyle, linéaire, ramifié ou cyclique renfermant de 1 à 4 atomes de carbone, par exemple, un radical méthyle.

Parmi les composés préférés de l'invention, on peut citer les composés pour lesquels $R_3$ et $R_4$ représentent un atome d'hydrogène, ainsi que ceux pour lesquels Y représente un atome d'hydrogène ou un atome de fluor.

Comme composés préférés de l'invention, on peut citer les composés pour lesquels Z représente un atome d'oxygène et notamment les composés répondant à la formule :

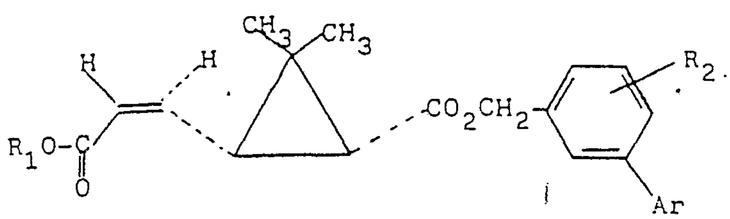

dans laquelle la copule cyclopropanique est de structure 1R, cis, la géométrie de la double liaison est Z, $R_1$, $R_2$, et Ar conservant les mêmes significations préférées que précédemment.

L'invention a également pour objet un procédé de préparation des composés de formule I, caractérisé en ce que l'on soumet un acide de formule :

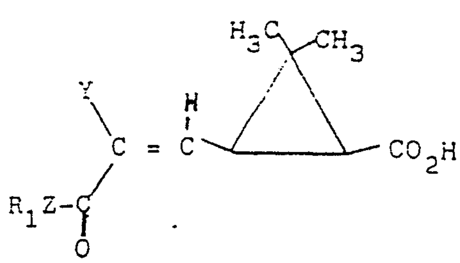

(II)

dans laquelle Y, $R_1$ et Z sont définis comme précédemment, ou un dérivé fonctionnel de cet acide, à l'action d'un alcool de formule III :

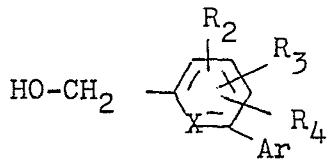

(III)

dans laquelle $R_2$, $R_3$, $R_4$, X et Ar sont définis comme précédemment, pour obtenir le composé de formule I correspondant.

L'estérification de l'acide II avec l'alcool III peut être effectuée en présence d'une base tertiaire, par exemple, en présence de pyridine ou de 4-diméthylamino pyridine et de dicyclohexyl carbodiimide.

Certains acides de formule II peuvent être préparés, par exemple, selon les procédés décrits dans les demandes de brevets européens n° 0038271, 0041021, 0048186 et 0050534.

Les acides de formule II dans laquelle Z représente un atome de soufre peuvent être préparés par exemple à partir des acides de formule II' :

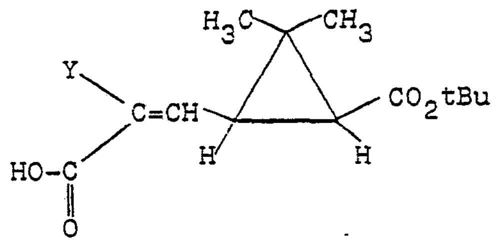

(II')

par action, au sein d'un solvant organique et en présence de dicyclohexylcarbodiimide, d'un mercaptan de formule :

$$R_1—SH$$

puis saponification de l'ester tert-butylique obtenu.

Les acides de formule II dans laquelle Y représente un radical O-alc ou

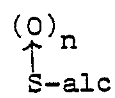

peuvent être préparés par exemple à partir des composés de formule II" :

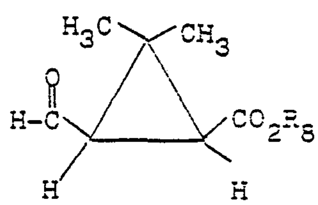

(II")

dans laquelle $R_8$ représente un atome d'hydrogène ou le reste d'un ester clivable, par action d'un composé de formule :

$$Y_1—CH_2—CO_2R_1$$

dans laquelle $Y_1$ a la valeur de Y indiquée ci-dessus, puis, le cas échéant, hydrolyse de l'ester clivable.

Les alcools de formule III sont pour certains d'entre eux, des produits connus ; ils peuvent être préparés selon les procédés décrits dans la demande de brevet PCT WO 82/01368, ou encore dans la demande de brevet européen n° 0049977.

La préparation de certains alcools nouveaux de formule III figure ci-après dans la partie expérimentale.

Les composés de formule (I) présentent d'intéressantes propriétés qui permettent leur utilisation dans la lutte contre les parasites. Il peut s'agir, par exemple, de la lutte contre les parasites des végétaux, les parasites des locaux et les parasites des animaux à sang chaud.

C'est ainsi que l'on peut utiliser les produits de formule I pour lutter contre les insectes, les nématodes et les acariens parasites des végétaux et des animaux.

L'invention a donc pour objet les composés de formule (I) pour leur utilisation à la lutte contre les parasites des végétaux, les parasites des locaux et les parasites des animaux à sang chaud.

L'invention a donc également pour objet les compositions pesticides destinées à la lutte contre les

parasites des végétaux, les parasites des locaux et les parasites des animaux à sang chaud, caractérisées en ce qu'elles renferment comme principe actif, au moins un composé défini précédemment.

Dans les compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensioactif, non ionique assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les produits de formule (I) peuvent être utilisés notamment pour lutter contre les insectes dans le domaine agricole, pour lutter, par exemple, contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) peuvent également être utilisés pour lutter contre les insectes parasites des animaux, par exemple, les poux notamment chez les bovins, les ovins et les volailles.

L'invention a donc notamment pour objet les compositions insecticides renfermant comme principe actif au moins l'un des composés définis précédemment.

Les composés de l'invention peuvent également être utilisés pour lutter contre les acariens et les nématodes parasites des végétaux.

L'invention a donc également pour objet les compositions acaricides ainsi que les compositions nématicides renfermant comme principe actif, au moins un composé de formule I.

Les compositions insecticides selon l'invention contiennent de préférence de 0,005 % à 10 % en poids de matière active.

Selon un mode opératoire avantageux, pour un usage dans les locaux, les compositions insecticides selon l'invention sont utilisées sous forme de compositions fumigantes.

Les compositions insecticides selon l'invention peuvent alors être avantageusement constituées, pour la partie non active, d'un serpentin combustible ou encore d'un substrat fibreux incombustible. Dans ce dernier cas, le fumigant obtenu après incorporation de la matière active est placé sur un appareil chauffant tel qu'un électro-émanageur.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 % à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions acaricides et nématicides peuvent se présenter notamment sous forme de poudres, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Les composés de formule (I) peuvent encore être utilisés pour lutter contre les acariens parasites des animaux, pour lutter, par exemple, contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions utilisées dans la lutte contre les acariens parasites des animaux à sang chaud, caractérisées en ce qu'elles renferment au moins un produit défini ci-dessus.

Ces compositions peuvent être administrées par voie externe, par vaporisation, par shampooing, par bain ou badigeonnage. Elles peuvent être également administrées par badigeonnage de l'épine dorsale selon la méthode dite méthode « pour on ». Elles peuvent être également administrées par voie digestive.

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple, des farines de poissons, des acides aminés de synthèse, des sels minéraux, des vitamines et des anti-oxydants.

L'invention a ainsi également pour objet les compositions destinées à l'alimentation animale renfermant comme principe actif au moins l'un des produits définis précédemment.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo/2,2,1/ 5-heptèn-2,3-di-carboximide, ou le pipéronyl-bis-2-(2'n-butoxy éthoxy) éthylacétal (ou tropital).

L'invention a également pour objet les associations douées d'activité insecticide, acaricide ou nématicide, caractérisées en ce qu'elles contiennent comme matière active, d'une part, un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichloro-vinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzylique des acides 2,2-diméthyl 3-(1,2,2,2-tétrahaloéthyl) cyclopropane-1-carboxyliques, dans lesquels « halo » représente un atome de fluor, de chlore ou de brome, étant entendu que les composés I peuvent exister sous toutes leurs formes stéréoisomères possibles de même que les copules acides et alcools des esters pyréthrinoïdes ci-dessus.

L'invention a enfin pour objet, à titre de produits industriels nouveaux et, notamment à titre de produits intermédiaires nécessaires à la préparation des produits de formule I, les alcools de formule III suivants :

le 2-(pyrrol-1-yl) 6-pyridyl méthanol ;

le (R, S) cyano/2-méthyl 3-(pyrrol-1-yl) phényl/méthanol.

Les exemples suivants illustrent l'invention sans toutefois la limiter.

Exemple 1 (1R, cis Z) 2,2-diméthyl-3-/(3-méthoxy 3-oxo) 1-propényl/cyclopropane carboxylate de /2-méthyl 3-(pyrrol-1-yl) phényl/méthyle.

On mélange 11 m.moles d'acide (1R, cis, Z) 2,2-diméthyl 3-/(3-méthoxy 3-oxo)-1-propényl cyclopropane carboxylique, 10 volumes de chlorure de méthylène, 10 m.moles d'alcool /2-méthyl 3-(pyrrol-1-yl)/phényl méthylique et 11 m.moles de dicyclohexylcarbodiimide. On refroidit à 0°, + 5 °C sous agitation et azote. On ajoute 0,5 % en poids par rapport à l'acide de diméthylamino pyridine. On maintient sous agitation et azote en laissant remonter à la température ambiante soit environ 4 heures. On essore le précipité, rince au chlorure de méthylène et distille sous pression réduite. On purifie le produit brut par chromatographie sur silice en éluant par le mélange hexane-éther isopropylique (8-2). On obtient le produit recherché. Rendement = 83 %.

$/\alpha/_D = + 47°$ (c = 0,6 % $CHCl_3$).

Exemples 2 à 20

En opérant comme à l'exemple 1, à partir des acides et des alcools correspondants, décrits dans la littérature, ou dont la préparation est fournie ci-après, on a obtenu les produits, répondant à la formule I :

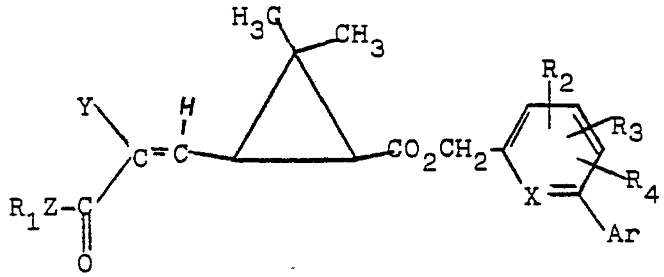

dont les noms suivent :

Exemple 2

(1R, cis, Z) 2,2-diméthyl 3-/(3-méthoxy 3-oxo) 1-propenyl/cyclopropane carboxylate de /(2-méthyl 3-phényl) phényl/méthyle ;

Exemple 3

(1R, cis, Z) 2,2-diméthyl 3-/(3-tertbutoxy 3-oxo) 1-propényl/cyclopropane carboxylate de //2-méthyl 3-(pyrrol-1-yl) phényl/méthyle ;

Exemple 4

(1R, cis, Z) 2,2-diméthyl 3-/(3-tertbutoxy 3-oxo) 1-propényl/cyclopropane carboxylate de /(2-méthyl 3-phényl) phényl/méthyle ;

Exemple 5

(1R, cis, E) 2,2-diméthyl 3-/(3-éthoxy 3-oxo 2-fluoro) 1-propényl/cyclopropane carboxylate de //2-méthyl 3-(pyrrol-1-yl) phényl/méthyle ;

Exemple 6

(1R, cis, E) 2,2-diméthyl 3-/(3-éthoxy 3-oxo 2-fluoro) 1-propényl/cyclopropane carboxylate de /(2-méthyl 3-phényl) phényl/méthyle ;

Exemple 7

(1R, cis, E) 2,2-diméthyl 3-/(3-tertbutoxy 3-oxo 2-fluoro) 1-propényl/cyclopropane carboxylate de /(2-méthyl 3-phényl) phényl/méthyle ;

Exemple 8

(1R, cis, E) 2,2-diméthyl 3-/(3-tertbutoxy 3-oxo 2-fluoro) 1-propényl/cyclopropane carboxylate de //2-méthyl 3-(pyrrol-1-yl) phényl/méthyle ;

Exemple 9

(1R, cis, E) 2,2-diméthyl 3-(3-éthoxy 3-oxo 2-fluoro) 1-propényl/cyclopropane carboxylate de //2-méthyl 3-(2,5-diméthyl pyrrol-1-yl) phényl/méthyle ;

Exemple 10

(1R, cis, Z) 2,2-diméthyl 3-/(3-tertbutoxy 3-oxo) 1-propényl/cyclopropane carboxylate de //2-méthyl 3-(2,5-diméthyl pyrrol-1-yl) phényl/méthyle ;

Exemple 11

(1R, cis, Z) 2,2-diméthyl 3-/(3-méthoxy 3-oxo) 1-propényl/cyclopropane carboxylate de //2-méthyl 3-(2,5-diméthyl pyrrol-1-yl) phényl/méthyle ;

Exemple 12

(1R, cis, Z) 2,2-diméthyl 3-/(3-méthoxy 3-oxo) 1-propényl/cyclopropane carboxylate de /3-(pyrrol-1-yl) phényl/méthyle ;

Exemple 13

(1R, cis, Z) 2,2-diméthyl 3-(3-tertbutoxy 3-oxo) 1-propényl/cyclopropane carboxylate de /3-(pyrrol-1-yl) phényl/méthyle ;

Exemple 14

(1R, cis, Z) 2,2-diméthyl 3-/(3-méthoxy 3-oxo) 1-propényl/cyclopropane carboxylate de //4-méthyl 3-(pyrrol-1-yl) phényl/méthyle ;

Exemple 15

(1R, cis, Z) 2,2-diméthyl 3-/(3-tertbutoxy 3-oxo) 1-propényl/cyclopropane carboxylate de //4-méthyl 3-(pyrrol-1-yl) phényl/méthyle ;

Exemple 16

(1R, cis, Z) 2,2-diméthyl 3-/(3-méthoxy 3-oxo) 1-propényl/cyclopropane carboxylate de //2,4-diméthyl 3-(pyrrol-1-yl) phényl/méthyle ;

Exemple 17

(1R, cis, Z) 2,2-diméthyl 3-/(3-tertbutoxy 3-oxo) 1-propényl/cyclopropane carboxylate de //2,4-diméthyl 3-(pyrrol-1-yl) phényl/méthyle ;

Exemple 18

(1R, cis, Z) 2,2-diméthyl 3-(3-tertbutoxy 3-oxo) 1-propényl/cyclopropane carboxylate de /6-(pyrrol-1-yl) 2-pyridyl/méthyle ;

Exemple 19

(1R, cis, Z) 2,2-diméthyl 3-/(3-méthoxy 3-oxo) 1-propényl/cyclopropane carboxylate de /6-(pyrrol-1-yl) 2-pyridyl/méthyle ;

Exemple 20

(1R, cis, Z) 2,2-diméthyl 3-/(3-tertbutoxy 3-oxo) 1-propényl/cyclopropane carboxylate de (R) et de (S) - cyano //2-méthyl 3-(pyrrol-1-yl)/phényl/méthyle.

Les principales conditions opératoires et les constantes des produits obtenus sont rapportées dans les tableaux ci-après :

(Voir Tableau page 11 et suivantes)

| | Y | Z | $R_1$ | 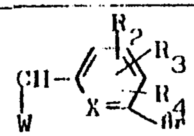 | Temps de réaction | Rendement | $\alpha_D (CHCl_3)$ | Solvant Chromatographie |
|---|---|---|---|---|---|---|---|---|
| Exemple 1 | H | O | $CH_3$ | 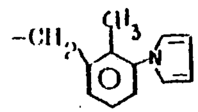 | 4 h | 83 % | + 47° c = 0,6 % | hexane éther isopropylique (8-2) |
| Exemple 2 | H | O | $CH_3$ | 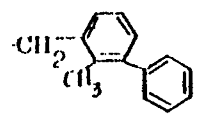 | 20 h | 85 % | + 39° c = 0,5 % | hexane éther isopropylique (9-1) |
| Exemple 3 | H | O | 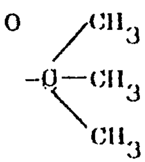 | 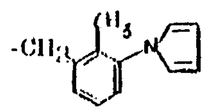 | 5 h | 88 % | + 50° c = 0,55 % | hexane éther isopropylique (9-1) |
| Exemple 4 | H | O | 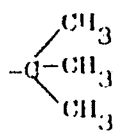 | 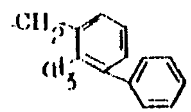 | 5 h | 86 % | + 46,5° c = 0,3 % | hexane éther isopropylique ( 9-1) |
| Exemple 5 | F | O | $C_2H_5$ | 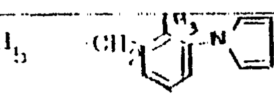 | 1 h 45 | 82 % | + 20° c = 0,5 % | hexane éther isopropylique (85-15) |
| Exemple 6 | F | O | $C_2H_5$ | 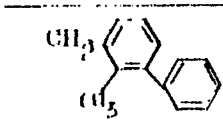 | 3 h | 78 % | +16,5° c = 0,5 % | hexane éther isopropylique (8-2) |

| | Y | Z | $R_1$ | 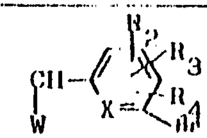 | Temps de réaction | Rendement | $\alpha_D(CHCl_3)$ | Solvant Chromatographie |
|---|---|---|---|---|---|---|---|---|
| Exemple 7 | F | O | 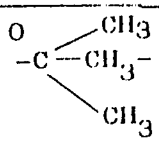 | 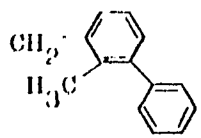 | 17 H | 85 % | +25° ± 2° c = 0,4 % | hexane éther isopropylique (9-1) |
| Exemple 8 | F | O | 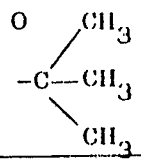 | 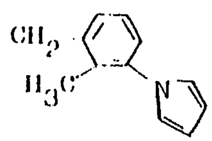 | 17 H | 89 % | +30,5° ±2° c = 0,5 % | hexane éther isopropylique (9-1) |
| Exemple 9 | F | O | $-C_2H_5$ | 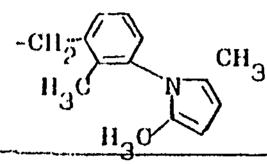 | 2 H | 68 % | +26° ± 2° c = 0,5 % | hexane éther isopropylique (7-3) |
| Exemple 10 | H | O | 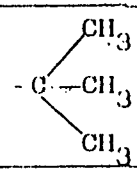 | " | 15 H | 77 % | +49,5° ± 2,5° c = 0,5 % | hexane Acétate d'éthyle (9-1) |
| Exemple 11 | H | O | $CH_3$ | " | 16 H | 83 % | +54,5°±2,5° c = 0,5 % | hexane Acétate d'éthyle (9,5-0,5) |
| Exemple 12 | H | O | $CH_3$ | 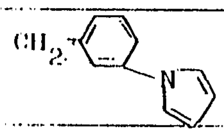 | 18 H | 78 % | +51,0° ±1,5° c = 0,8 % | hexane Acétate d'éthyle (8-2) |

| | Y | Z | $R_1$ | 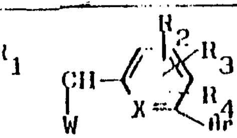 | Temps de réaction | Rendement | $\alpha_D(CHCl_3)$ | Solvant Chromatographie |
|---|---|---|---|---|---|---|---|---|
| Exemple 13 | H | O | 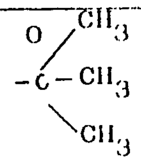 | 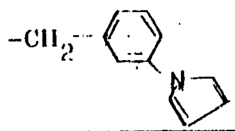 | 18 H | 66 % | +58,5°±1,5° c = 1 % F = 82°C | hexane Acétate d'éthyle (9-1) |
| Exemple 14 | H | O | $CH_3$ | 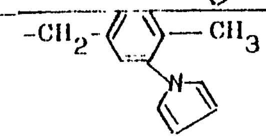 | 3 H | 90 % | +44° ± 2° c = 0,5 % | hexane Acétate d'éthyle (9-1) |
| Exemple 15 | H | O | 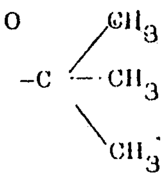 | Idem exemple 14 | 3 H | 82 % | +48° ± 2° c = 0,5 % | hexane Acétate d'éthyle (9-1) |
| Exemple 16 | H | O | $CH_3$ | 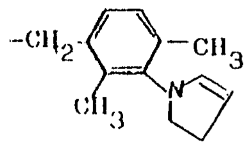 | 2 H | 62 % | +37° ± 2° c = 0,5 % | hexane Acétate d'éthyle (9-1) |
| Exemple 17 | H | O | 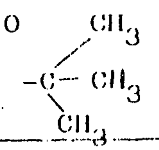 | Idem exemple 16 | 2 H | 86 % | +42,5° ±2° c = 0,5 % | hexane Acétate d'éthyle (9-1) |
| Exemple 18 | H | O | " | 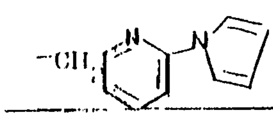 | 1 H 30 | 79 % | +99,5° ±0,5° c = 0,5 % | hexane Acétate d'éthyle (8-2) |

| | Y | Z | $R_1$ | 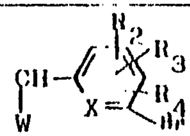 | Temps de réaction | Rendement | $\alpha_D(CHCl_3)$ | Solvant Chromatographie |
|---|---|---|---|---|---|---|---|---|
| Exemple 19 | H | O | $CH_3$ | 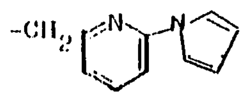 | 1 H 30 | 61 % | +87,5° ± 1,5°<br>c = 1 %<br>F = 80°C | hexane<br>Acétate d'éthyle<br>(8-2) |
| Exemple 2O | H | O | $CH_3$<br>C $CH_3$<br>$CH_3$ | 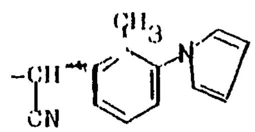<br>(R et S) | 15 H | 22 %<br>(isomère R ou S)<br>9,3%<br>(isomère S ou R) | Analyse :<br>$C_{26}H_{30}N_2O_4$<br>calculé :<br>C % 71,87<br>H % 6,96<br>N % 6,45<br>Trouvé :<br>C % 72,0<br>H % 7,1<br>N % 6,3 | hexane<br>Ether isopropylique<br>(7-3) |

Préparation du (R, S) cyano/2-méthyl 3-(pyrrol-1-yl) phényl/méthanol.

A. 3-(pyrrol-1-yl) 2-méthyl benzaldéhyde.

On agite pendant 3 heures à température ambiante, une suspension comprenant 6 g de 3-(pyrrol-1-yl) 2-méthyl phényl méthanol 300 $cm^3$ de benzène et 30 g de bioxyde de manganèse.

On ajoute 12 g de bioxyde de manganèse, agite deux heures et ajoute de nouveau 6 g de bioxyde de manganèse. Après 19 heures, d'agitation à température ambiante, on essore, rince au benzène, concentre à sec le filtrat sous pression réduite et obtient 4,89 g de produit attendu. F = 82 °C.

B. (R, S) cyano/2-méthyl 3-(pyrrol-1-yl) phényl/méthanol.

Dans une solution refroidie à + 10 °C comprenant 4,89 g du produit obtenu au stade A, 50 $cm^3$ de méthanol et 20 $cm^3$ d'eau, on ajoute goutte à goutte, 4 $cm^3$ d'acide acétique puis 1,4 g de cyanure de sodium, agite 5 heures à + 10 °C et verse dans 60 $cm^3$ d'eau glacée. On agite une heure, décante, extrait au chlorure de méthylène, sèche et concentre à sec sous pression réduite. On chromatographie le résidu sur silice (éluant hexane-acétate d'éthyle 8-2) et recueille 3,59 g de produit attendu.

Spectre de RMN $CDCl_3$

5.78 ppm

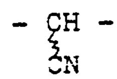

2,23 ppm

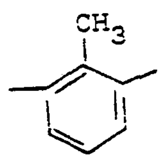

Préparation de 2-(pyrrol-1-yl) 6-pyridyl méthanol.

A. 6-acétyl amino 2-méthyl pyridine.

On chauffe une heure au reflux 50 g de 6-amino 2-méthyl pyridine dans 250 $cm^3$ d'anhydride acétique. On concentre à sec sous pression réduite, reprend le résidu au chlorure de méthylène, lave à l'eau puis avec une solution aqueuse de soude, puis à l'eau, sèche et concentre à sec sous pression réduite. On obtient une huile qui cristallise dans l'éther isopropylique.

On recueille 41 g de produit attendu. F = 87 °C.

B. Acide 6-acétylamino picolinique.

On chauffe à 70 °C, 40 g du produit obtenu au stade A dans 400 $cm^3$ d'eau et ajoute en deux heures, 100 g de permanganate de potassium, on agite une heure trente à 70-80 °C, filtre la solution chaude, concentre sous pression réduite jusqu'au tiers du volume.

On refroidit à 10 °C/15 °C, élimine par filtration le produit cristallisé, acidifie la phase aqueuse à l'aide d'acide chlorhydrique 2N, essore le produit cristallisé, empâte à l'éthanol, puis à l'éther et sèche sous pression réduite. Après recristallisation dans l'éthanol, on obtient 16 g de produit attendu. F = 212 °C.

C. 2-amino 6-méthoxy carbonyl pyridine.

On refroidit à + 5 °C, 15 g du produit préparé au stade B dans 150 $cm^3$ de méthanol et effectue un barbottage d'acide chlorhydrique pendant une heure à température ambiante puis pendant 4 heures à température du reflux.

On laisse revenir à température, agite pendant 16 heures, concentre à sec sous pression réduite, reprend le résidu par 50 $cm^3$ d'eau, filtre, alcalinise le filtrat par de l'ammoniaque concentrée, extrait au chloroforme, sèche et concentre à sec sous pression réduite. On obtient 9,54 g de produit attendu. F = 87 °C.

D. 2-(pyrrol-1-yl) 6-méthoxy carbonyl pyridine.

On dissout 7 g de produit préparé au stade C dans 17,5 $cm^3$ d'acide acétique par agitation à température ambiante puis ajoute 6,3 $cm^3$ de 2,5-diméthoxytétrahydrofurane. On chauffe une heure au reflux, élimine l'acide acétique sous pression réduite, purifie l'huile obtenue par chromatographie sur silice en éluant au chlorure de méthylène et recueille 3,7 g de produit attendu. F = 61 °C.

E. 2-(pyrrol-1-yl) 6-pyridyl méthanol.

On refroidit à – 40 °C, 3,7 g de produit obtenu au stade D dans 40 $cm^3$ de toluène et ajoute goutte à goutte en une heure 38 $cm^3$ de diisobutyl aluminium.

On agite 30 minutes à – 30 °C puis sans que la température ne dépasse + 10 °C, on ajoute lentement, 110 $cm^3$ d'une solution aqueuse molaire de tartrate double de sodium et de potassium. On agite 16 heures à température ambiante, décante, sèche et concentre à sec sous pression réduite. On obtient 2,9 g de produit attendu. F = 55 °C.

Exemples de compositions :

Exemple A : Préparation d'un concentré soluble

On effectue un mélange homogène de :

| | |
|---|---|
| Produit de l'exemple 1 | 0,25 g |
| Butoxyde de pipéronyle | 1 g |
| Tween 80 | 0,25 g |
| Topanol A | 0,1 g |
| Eau | 98,4 g |

Exemple B : Préparation d'un concentré émulsifiable

On mélange intimement :

| | |
|---|---|
| Produit de l'exemple 2 | 0,015 g |
| Butoxyde de pipéronyle | 0,5 g |
| Topanol A | 0,1 g |
| Xylène | 95,885 g |
| Tween 80 | 3,5 g |

Exemple C : Préparation d'un concentré émulsifiable

On effectue un mélange homogène de :

| | |
|---|---|
| Produit de l'exemple 3 | 1,5 g |
| Tween 80 | 20 g |
| Topanol A | 0,1 g |
| Xylène | 78,4 g |

Etude de l'activité des composés selon l'invention sur les parasites.

a) Etude de l'effet létal des composés de l'invention sur mouches domestiques.

Les insectes tests sont des mouches domestiques femelles de souche sensible aux pyréthrinoïdes élevées à 22-23 °C et 60-65 % d'humidité relative et âgées de 4 à 5 jours. On opère par application topique de 1 µl de solution acétonique sur le thorax dorsal des insectes à l'aide du micro-manipulateur d'Arnold. On utilise 50 individus par dose du produit à tester. On effectue le contrôle de mortalité vingt-quatre heures après traitement.

On constate que dans le test utilisé les produits présentent une bonne activité létale, notamment les produits des exemples 5 et 6, pour lesquels la DL 50 en ng/insecte a été trouvée respectivement de 12 et 5,1.

b) Etude de l'effet létal des composés de l'invention sur larves de Spodoptera Littoralis.

Les essais sont effectués par application topique d'une solution acétonique à l'aide du micro-manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24 °C et 65 % d'humidité relative. Après traitement, les individus sont placés sur un milieu nutritif artificiel (milieu de Poitout).

On effectue le contrôle des mortalités 48 heures après traitement.

On constate que dans le test utilisé, les produits présentent une bonne activité létale, notamment ceux des exemples 1 et 2, pour lesquels la DL 50 en ng/insecte a été trouvée respectivement de 6,9 et 7,1.

c) Etude de l'activité des produits de l'invention sur larves de Acanthocephalus Obtectus.

Les essais sont effectués par application topique de manière analogue à celle utilisée pour les mouches et les larves de Spodoptera. On utilise des larves de l'avant-dernier stade larvaire et après traitement, les larves sont alimentées par des plants de haricots. On effectue le contrôle de mortalité 72 heures après traitement.

On constate que dans le test utilisé, les produits présentent une bonne activité létale, notamment celui de l'exemple 6, dont la DL 50 a été de 12,1 ng/insecte.

d) Etude de l'activité de choc sur mouche domestique.

Les insectes tests sont des mouches domestiques femelles âgées de 4 à 5 jours. On opère par pulvérisation directe en cylindre de Kearns et March en utilisant comme solvant un mélange d'acétone (5 %) et d'Isopar L (solvant pétrolier) (quantité de solvant utilisée 2 ml en une seconde). On utilise 50 insectes par dose. On effectue les contrôles toutes les minutes jusqu'à 10 minutes, puis à 15 minutes et l'on détermine le KT 50 par les méthodes habituelles.

On constate que les produits présentent une bonne activité.

Les résultats sont résumés dans le tableau suivant :

| Produit de l'exemple | KT 50 mn |
|---|---|
| 1 | 3,54 |
| 2 | 4,1 |
| 5 | 3,54 |
| 12 | 3,19 |
| 14 | 3,16 |

e) Activité sur Tétranychus Urticae

Essai adulticide

On utilise des plants de haricots comportant deux feuilles qui sont traitées au pistolet Fisher à différentes doses des produits à tester. Après séchage, ces plants sont infestés à raison de 25 femelles de Tétranychus Urticae par feuille et maintenus à 22-23 °C, 60-65 % d'humidité relative artificielle permanente. Les dénombrements des acariens vivants et morts sont effectués 24 heures et 48 heures après traitement.

Les produits de l'invention présentent une bonne activité adulticide dans ce test.

Les résultats sont résumés dans le tableau suivant :

| Produit de l'exemple | CL 50 en mg/hl |
|---|---|
| 3 | 419 |
| 4 | 476 |
| 7 | 399 |
| 8 | 661 |

**Revendications** (pour les Etats contractants : BE, CH, DE, FR, GB, IT, LI, LU, NL, SE)

1. Sous toutes les formes d'isomères possibles ou sous forme de mélanges d'isomères, les composés de formule (I) :

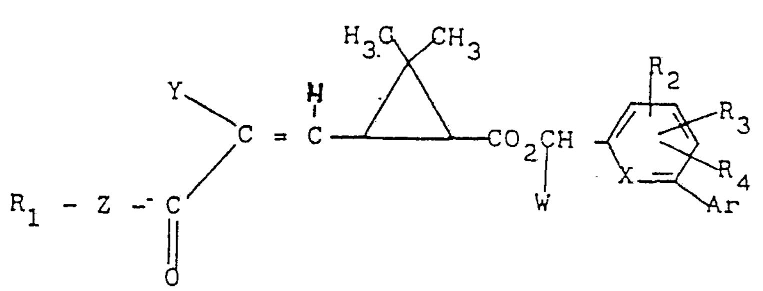

(I)

dans laquelle Y représente un atome d'hydrogène ou d'halogène ou un radical —Oalc ou

-S-alc
↓
$(O)_n$

dans lequel alc est un radical alcoyle renfermant de 1 à 6 atomes de carbone et n est égal à 0, 1 ou 2, Z représente un atome d'oxygène ou de soufre, $R_1$ représente

ou bien un radical alcoyle, linéaire, ramifié ou cyclique, saturé ou insaturé, renfermant de 1 à 8 atomes de carbone, éventuellement substitué par un ou plusieurs groupements fonctionnels, identiques ou différents,

ou bien un groupement aryle renfermant de 6 à 14 atomes de carbone, éventuellement substitué par un ou plusieurs groupements fonctionnels, identiques ou différents,

ou bien un radical hétérocyclique éventuellement substitué par un ou plusieurs groupements fonctionnels, identiques ou différents, $R_2$, $R_3$ et $R_4$, identiques ou différents, représentent un atome d'hydrogène, un atome d'halogène, un radical alcoyle renfermant jusqu'à 8 atomes de carbone, un radical haloalcoyle renfermant jusqu'à 8 atomes de carbone pouvant renfermer plusieurs atomes d'halogène, un radical alcoxy renfermant jusqu'à 8 atomes de carbone, W représente un atome d'hydrogène ou un radical cyano, X représente un atome de carbone ou un atome d'azote, Ar représente un radical phényle, pyridyle, thiényle, naphtyle, furyle ou pyrrolyle éventuellement substitué et la double liaison éthylénique à la géométrie E ou Z.

2. Les composés de formule I, tels que définis à la revendication 1, pour lesquels Y représente un atome d'hydrogène ou d'halogène, $R_2$, $R_3$ et $R_4$ sont identiques, W représente un atome d'hydrogène, X représente un atome de carbone.

3. Les composés de formule I tels que définis à la revendication 1 ou 2, pour lesquels la structure de la copule cyclopropanique est 1R, cis.

4. Les composés de formule I tels que définis à la revendication 1, 2 ou 3, pour lesquels Ar représente un radical pyrrolyle.

5. Les composés de formule I tels que définis à la revendication 1, 2 ou 3, pour lesquels Ar représente un radical phényle.

6. Les composés de formule I tels que définis à l'une quelconque des revendications 1 à 5, pour lesquels un ou plusieurs des radicaux, $R_2$, $R_3$ et $R_4$ représentent un radical alcoyle, linéaire, ramifié ou cyclique renfermant de 1 à 4 atomes de carbone.

7. Les composés de formule I tels que définis à la revendication 6, pour lesquels un ou plusieurs des radicaux, $R_2$, $R_3$ et $R_4$ représentent un radical méthyle.

8. Les composés de formule I tels que définis à l'une quelconque des revendications 1 à 7, pour lesquels $R_3$ et $R_4$ représentent un atome d'hydrogène.

9. Les composés de formule I tels que définis à l'une quelconque des revendications 1 à 8, pour lesquels Y représente un atome d'hydrogène.

10. Les composés de formule I tels que définis à l'une quelconque des revendications 1 à 8, pour lesquels Y représente un atome de fluor.

11. Les composés de formule I tels que définis à l'une quelconque des revendications 1 à 10, pour lesquels Z représente un atome d'oxygène.

12. Les composés de formule I tels que définis à l'une quelconque des revendications 1 à 9 et 11, répondant à la formule :

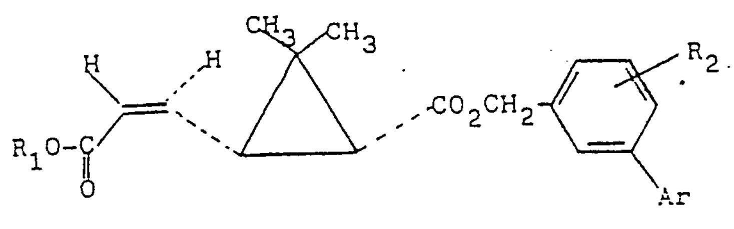

dans laquelle la copule cyclopropanique est de structure 1R, cis la géométrie de la double liaison est Z, $R_1$, $R_2$ et Ar conservant la même signification que dans la revendication 1.

13. Procédé de préparation des composés de formule I tels que définis à l'une quelconque des revendications 1 à 12 ; caractérisé en ce que l'on soumet un acide de formule :

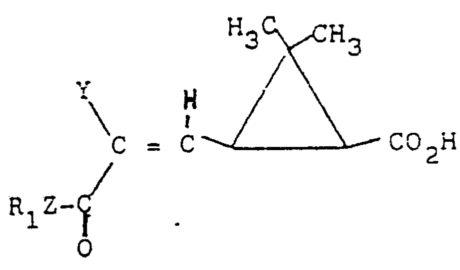

(II)

dans laquelle Y, $R_1$ et Z sont définis comme dans la revendication 1, ou un dérivé fonctionnel de cet acide, à l'action d'un alcool de formule III :

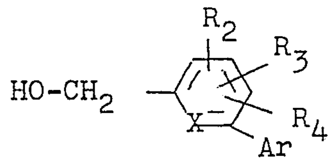

(III)

dans laquelle $R_2$, $R_3$, $R_4$, X et Ar sont définis comme dans la revendication 1, pour obtenir le composé de formule I correspondant.

14. Les composés de formule I tels que définis à l'une quelconque des revendications 1 à 12, pour leur utilisation à la lutte contre les parasites des végétaux, les parasites des locaux et les parasites des animaux à sang chaud.

15. Les compositions pesticides destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 12.

16. Les compositions insecticides renfermant comme principe actif, au moins l'un des composés définis à l'une quelconque des revendications 1 à 12.

17. Les compositions acaricides destinées à lutter contre les acariens parasites des végétaux, renfermant comme principe actif au moins l'un des composés définis à l'une quelconque des revendications 1 à 12.

18. Les compositions nématicides renfermant comme principe actif, au moins l'un des composés définis à l'une quelconque des revendications 1 à 12.

19. Les compositions acaricides destinées à lutter contre les acariens parasites des animaux, renfermant comme principe actif au moins l'un des composés définis à l'une quelconque des revendications 1 à 12.

20. Les compositions destinées à l'alimentation animale, caractérisées en ce qu'elles renferment comme principe actif au moins l'un des composés définis à l'une quelconque des revendications 1 à 12.

21. Associations douées d'activité insecticide, acaricide ou nématicide, caractérisées en ce qu'elles contiennent comme matière active, d'une part, un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcools 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcools 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels « halo » représente un atome de fluor, de chlore ou de brome, étant entendu que les composés I peuvent exister sous toutes leurs formes stéréoisomères possibles de même que les copules acides et alcools des esters pyréthrinoïdes ci-dessus.

22. A titre de produits industriels nouveaux, les produits de formule III dont les noms suivent :
le 2-(pyrrol-1-yl) 6-pyridyl méthanol ;
le (R, S) cyano /2-méthyl 3-(pyrrol-1-yl) phényl/méthanol.

**Revendications** (pour l'Etat contractant AT)

1. Procédé pour préparer sous toutes les formes d'isomères possibles ou sous forme de mélanges d'isomères, les composés de formule (I) :

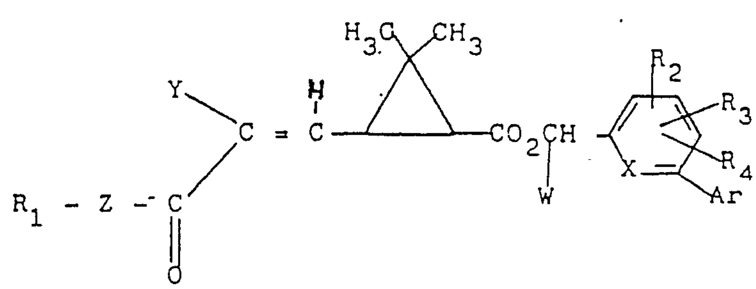

(I)

dans laquelle Y représente un atome d'hydrogène ou d'halogène ou un radical —Oalc ou

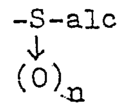

dans lequel alc est un radical alcoyle renfermant de 1 à 6 atomes de carbone et n est égal à 0, 1 ou 2,

Z représente un atome d'oxygène ou de soufre, $R_1$ représente

ou bien un radical alcoyle, linéaire, ramifié ou cyclique, saturé ou insaturé, renfermant de 1 à 8 atomes de carbone, éventuellement substitué par un ou plusieurs groupements fonctionnels, identiques ou différents,

ou bien un groupement aryle renfermant de 6 à 14 atomes de carbone, éventuellement substitué par un ou plusieurs groupements fonctionnels, identiques ou différents,

ou bien un radical hétérocyclique éventuellement substitué par un ou plusieurs groupements fonctionnels, identiques ou différents, $R_2$, $R_3$ et $R_4$, identiques ou différents, représentent un atome d'hydrogène, un atome d'halogène, un radical alcoyle renfermant jusqu'à 8 atomes de carbone, un radical haloalcoyle renfermant jusqu'à 8 atomes de carbone pouvant renfermer plusieurs atomes d'halogène, un radical alcoxy renfermant jusqu'à 8 atomes de carbone, W représente un atome d'hydrogène ou un radical cyano, X représente un atome de carbone ou un atome d'azote, Ar représente un radical phényle, pyridyle, thiényle, naphtyle, furyle ou pyrrolyle éventuellement substitué et la double liaison éthylénique a la géométrie E ou Z, caractérisé en ce que l'on soumet un acide de formule :

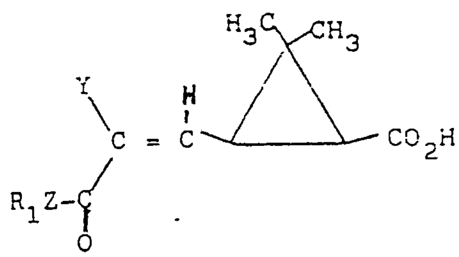

(II)

dans laquelle Y, $R_1$ et Z sont définis comme précédemment, ou un dérivé fonctionnel de cet acide, à l'action d'un alcool de formule III :

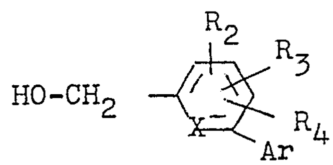

(III)

dans laquelle $R_2$, $R_3$, $R_4$, X et Ar sont définis comme précédemment pour obtenir le composé de formule I correspondant.

2. Procédé selon la revendication 1, caractérisé en ce que l'on utilise au départ un composé de formule II dans laquelle Y représente un atome d'hydrogène ou d'halogène et un composé de formule III dans laquelle $R_2$, $R_3$ et $R_4$ sont identiques, W représente un atome d'hydrogène et X représente un atome de carbone.

3. Procédé selon la revendication 2, caractérisé en ce que l'on utilise au départ un composé de formule II de structure 1R, cis.

4. Procédé selon la revendication 2 ou 3, caractérisé en ce que l'on utilise au départ un composé de formule III dans laquelle Ar représente un radical pyrrolyle.

5. Procédé selon la revendication 2 ou 3, caractérisé en ce que l'on utilise au départ un composé de formule III dans laquelle Ar représente un radical phényle.

6. Procédé selon l'une quelconque des revendications 2 à 5, caractérisé en ce que l'on utilise au départ un composé de formule III dans laquelle un ou plusieurs des radicaux $R_2$, $R_3$ et $R_4$ représentent un radical alcoyle, linéaire, ramifié ou cyclique renfermant de 1 à 4 atomes de carbone.

7. Procédé selon l'une quelconque des revendications 2 à 5, caractérisé en ce que l'on utilise au départ un composé de formule III dans laquelle un ou plusieurs des radicaux $R_2$, $R_3$ et $R_4$ représentent un radical méthyle.

8. Procédé selon l'une quelconque des revendications 2 à 5, caractérisé en ce que l'on utilise au départ un composé de formule III dans laquelle $R_3$ et $R_4$ représentent un atome d'hydrogène.

9. Procédé selon l'une quelconque des revendications 2 à 8, caractérisé en ce que l'on utilise au départ un composé de formule II dans laquelle Y représente un atome d'hydrogène.

10. Procédé selon l'une quelconque des revendications 2 à 8, caractérisé en ce que l'on utilise au départ un composé de formule II dans laquelle Y représente un atome de fluor.

11. Procédé selon l'une quelconque des revendications 2 à 10, caractérisé en ce que l'on utilise au départ un composé de formule II dans laquelle Z représente un atome d'oxygène.

12. Procédé selon l'une quelconque des revendications 2 à 9 et 11, caractérisé en ce que l'on utilise au départ un acide de formule $II_1$ :

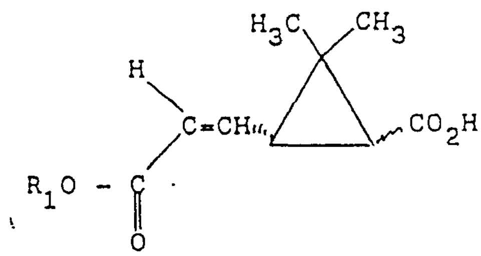

($II_1$)

de structure 1R,cis la géométrie de la double liaison étant Z et $R_1$ étant défini comme précédemment ou un dérivé fonctionnel de cet acide et un composé de formule $III_1$ :

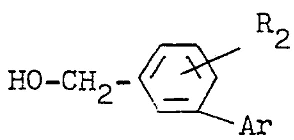

($III_1$)

dans laquelle $R_2$ et Ar sont définis comme précédemment.

13. Les compositions pesticides destinées à la lutte contre les parasites des végétaux, les parasites des locaux et les parasites des animaux à sang chaud, caractérisées en ce qu'elles renferment comme principe actif au moins un compsoé défini à l'une quelconque des revendications 1 à 12.

14. Les compositions insecticides renfermant comme principe actif, au moins l'un des composés définis à l'une quelconque des revendications 1 à 12.

15. Les compositions acaricides destinées à lutter contre les acariens parasites des végétaux, renfermant comme principe actif au moins l'un des composés définis à l'une quelconque des revendications 1 à 12.

16. Les compositions nématicides renfermant comme principe actif, au moins l'un des composés définis à l'une quelconque des revendications 1 à 12.

17. Les compositions acaricides destinées à lutter contre les acariens parasites des animaux, renfermant comme principe actif au moins l'un des composés définis à l'une quelconque des revendications 1 à 12.

18. Les compositions destinées à l'alimentation animale, caractérisées en ce qu'elles renferment comme principe actif au moins l'un des composés définis à l'une quelconque des revendications 1 à 12.

19. Compositions douées d'activité insecticide, acaricide ou nématicide, caractérisées en ce qu'elles contiennent comme matière active, d'une part, un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcools 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcools 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels « halo » représente un atome de fluor, de chlore ou de brome, étant entendu que les composés I peuvent exister sous toutes leurs formes stéréoisomères possibles de même que les copules acides et alcools des esters pyréthrinoïdes ci-dessus.

20. Les compositions telles que définies à l'une quelconque des revendications 13 à 19, caractérisées en ce qu'elles renferment à titre de principe actif, l'un au moins des produits de formule I, telle que définie à la revendication 2.

**Claims** (for the Contracting States : BE, CH, DE, FR, GB, IT, LI, LU, NL, SE)

1. In all the possible isomeric forms or in the form of a mixture of isomers, the compounds with the formula (I) :

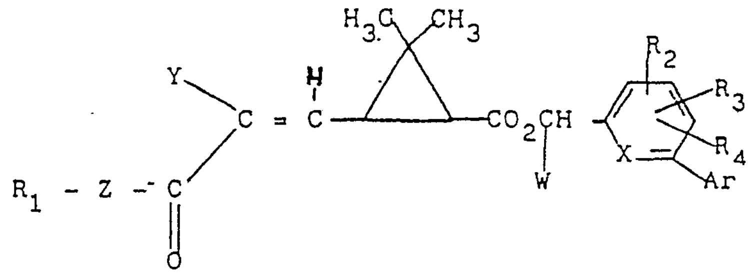

(I)

in which Y represents a hydrogen or a halogen atom or a radical —Oalk or

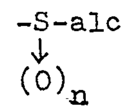

in which alk is an alkyl radical containing from 1 to 6 carbon atoms and n is 0, 1 or 2,

Z represents an oxygen or sulphur atom, $R_1$ represents

either a linear, branched or cyclic, saturated or unsaturated alkyl radical, containing from 1 to 8 carbon atoms, possibly substituted by one or more identical or different functional groups,

or an aryl group containing from 6 to 14 carbon atoms, possibly substituted by one or more identical or different functional groups,

or a heterocyclic radical possibly substituted by one or more identical or different functional groups, $R_2$, $R_3$ and $R_4$, being identical or different, represent a hydrogen atom, a halogen atom, an alkyl radical containing up to 8 carbon atoms, a haloalkyl radical containing up to 8 carbon atoms able to contain several halogen atoms, an alkoxy radical containing up to 8 carbon atoms, W represents a hydrogen atom or a cyano radical, X represents a carbon atom or a nitrogen atom, Ar represents a possibly-substituted phenyl, pyridyl, thienyl, naphthyl, furyl or pyrrolyl radical and the double ethylene bond has the geometry E or Z.

2. The compounds with the formula I, as defined in claim 1, for which Y represents a hydrogen or halogen atom, $R_2$, $R_3$ and $R_4$ are identical, W represents a hydrogen atom, X represents a carbon atom.

3. Compounds with the formula I as defined in claim 1 or 2, for which the cylopropane copula is of 1R,cis structure.

4. Compounds with the formula I as defined in claims 1, 2 or 3, for which Ar represents a pyrrolyl radical.

5. Compounds with the formula I as defined in claims 1, 2 or 3, for which Ar represents a phenyl radical.

6. Compounds with the formula I as defined in any one of the claims 1 to 5, for which one or more of the radicals, $R_2$, $R_3$ and $R_4$ represent a linear, branched or cyclic alkyl radical containing from 1 to 4 carbon atoms.

7. Compounds with the formula I as defined in claim 6, for which one or more of the radicals $R_2$, $R_3$ and $R_4$ represent a methyl radical.

8. Compounds with the formula I as defined in any one of the claims 1 to 7, for which $R_3$ and $R_4$ represent a hydrogen atom.

9. Compounds with the formula I as defined in any one of the claims 1 to 8, for which Y represents a hydrogen atom.

10. Compounds with the formula I as defined in any one of the claims 1 to 8, for which Y represents a fluorine atom.

11. Compounds with the formula I as defined in any one of the claims 1 to 10, for which Z represents an oxygen atom.

12. Compounds with the formula I as defined in any one of the claims 1 to 9 and 11, answering to the formula :

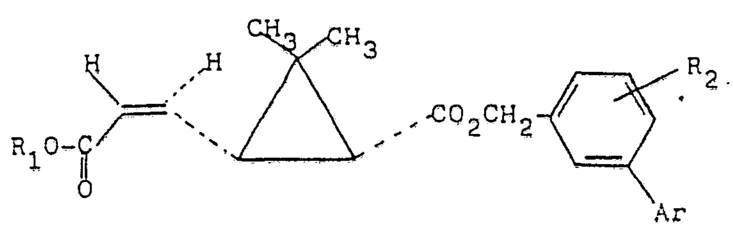

in which the cyclopropane copula is of 1R,cis structure, the geometry of the double bond is Z, $R_1$, $R_2$ and Ar retain the same significance as in claim 1.

13. Preparation process for compounds with the formula I as defined in any one of the claims 1 to 12, characterized in that an acid with the formula :

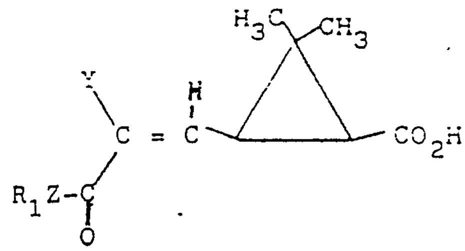

(II)

in which Y, $R_1$ and Z are defined as in claim 1, or a functional derivative of this acid, is submitted to the action of an alcohol with the formula III :

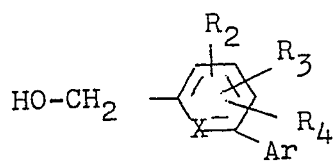

(III)

in which $R_2$, $R_3$, $R_4$, X and Ar are defined as in claim 1, so as to obtain the corresponding compound with the formula I.

14. Compounds with the formula I as defined in any one of the claims 1 to 12, for their use in combatting parasites of vegetation, parasites of premises and parasites of warm-blooded animals.

15. Pesticide compositions intended to combat parasites or vegetation, parasites of premises and parasites of warm-blooded animals, characterized in that they contain as active principle at least one compound defined in any one of the claims 1 to 12.

16. Insecticide compositions containing as active principle, at least one of the compounds defined in any one of the claims 1 to 12.

17. Acaricide compositions intended to combat parasitic acarids of vegetation, containing as active principle at least one of the compounds defined in any one of the claims 1 to 12.

18. Nematocide compositions containing as active principle, at least one of the compounds defined in any one of the claims 1 to 12.

19. Acaricide compositions intended to combat parasitic acarids of animals, containing as active principle at least one of the compounds defined in any one of the claims 1 to 12.

20. Compositions intended for animal food-stuffs, characterized in that they contain as active principle at least one of the compounds defined in any one of the claims 1 to 12.

21. Combinations endowed with insecticide, acaricide or nematocide activity, characterized in that they contain as active material, on the one hand, one at least of the compounds with the general formula (I), and on the other hand, one at least of the pyrethrinoid esters chosen from the group constituted by the esters of allethrolones, of 3,4,5,6-tetrahydrophthalimidomethyl alcohol, of 5-benzyl-3-furyl methyl alcohol, of 3-phenoxybenzyl alcohol and of α-cyano-3-phenoxybenzyl alcohols with chrysanthemic acids, by the esters of 5-benzyl-3-furyl methyl alcohol with 2,2-dimethyl-3-(2-oxo-3-tetrahydrothiophenylidenemethyl) cyclopropane-1-carboxylic acids, by the esters of 3-phenoxybenzyl alcohol and of α-cyano-3-phenoxybenzyl alcohols with 2,2-dimethyl-3-(2,2-dichlorovinyl) cyclopropane-1-carboxylic acids, by the esters of α-cyano-3-phenoxybenzyl alcohols with 2,2-dimethyl-3-(2,2-dibromovinyl) cyclopropane-1-carboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furyl methyl alcohol, of 3-phenoxybenzyl alcohol and of α-cyano-3-phenoxybenzyl alcohols with 2,2-dimethyl-3-(1,2,2,2-tetrahaloethyl) cyclopropane-1-carboxylic acids, in which « halo » represents a fluorine, chlorine or bromine atom, it being understood that the compounds I can exist in all their possible stereoisomeric forms, as can also the acid and alcohol copulas of the above pyrethrinoid esters.

22. As new industrial products, products with the formula III the names of which follow :
2-(pyrrol-1-yl) 6-pyridyl methanol ;
(R, S) cyano [2-methyl-3-(pyrrol-1-yl) phenyl] methanol.

**Claims** (for the Contracting State AT)

1. Process for the preparation, in all the possible isomeric forms or in the form of a mixture of isomers, of the compounds with the formula (I) :

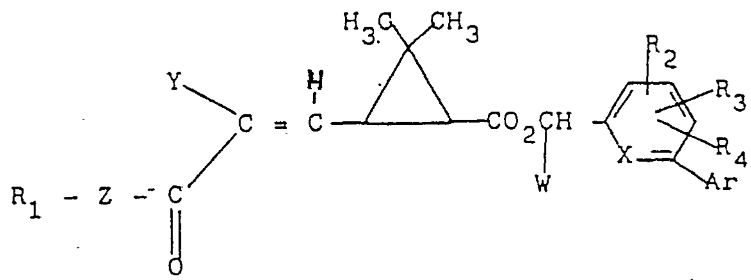

(I)

in which Y represents a hydrogen or a halogen atom or a radical —Oalk or

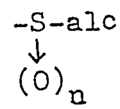

in which alk is an alkyl radical containing from 1 to 6 carbon atoms and n is 0, 1 or 2,

Z represents an oxygen or sulphur atom, $R_1$ represents

either a linear, branched or cyclic, saturated or unsaturated alkyl radical, containing from 1 to 8 carbon atoms, possibly substituted by one or more identical or different functional groups,

or an aryl group containing from 6 to 14 carbon atoms, possibly substituted by one or more identical or different functional groups,

or a heterocyclic radical possibly substituted by one or more identical or different functional groups, $R_2$, $R_3$ and $R_4$, being identical or different, represent a hydrogen atom, a halogen atom, an alkyl radical containing up to 8 carbon atoms, a haloalkyl radical containing up to 8 carbon atoms able to contain several halogen atoms, an alkoxy radical containing up to 8 carbon atoms, W represents a hydrogen atom or a cyano radical, X represents a carbon atom or a nitrogen atom, Ar represents a possibly-substituted phenyl, pyridyl, thienyl, naphthyl, furyl or pyrrolyl radical and the double ethylene bone has the geometry E or Z, characterized in that an acid with the formula :

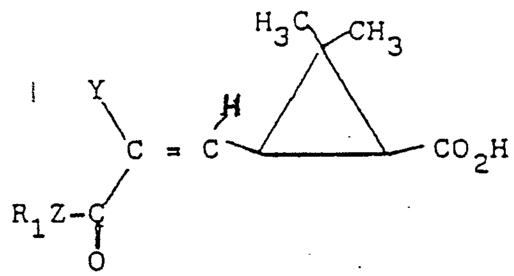

(II)

in which Y, $R_1$ and Z are defined as previously, or a functional derivative of this acid, is submitted to the action of an alcohol with the formula III :

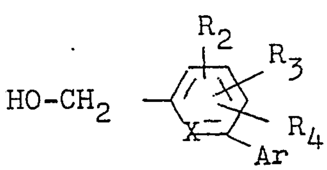

(III)

in which $R_2$, $R_3$, $R_4$, X and Ar are defined as previously so as to obtain the corresponding compound with the formula I.

2. Process according to claim 1, characterized in that there is used at the start a compound with the formula II in which Y represents a hydrogen or halogen atom and a compound with the formula III in which $R_2$, $R_3$ and $R_4$ are identical, W represents a hydrogen atom and X represents a carbon atom.

3. Process according to claim 2, characterized in that at the start a compound with the formula II is used with 1R, cis structure.

4. Process according to claim 2 or 3, characterized in that at the start a compound with the formula III is used in which Ar represents a pyrrolyl radical.

5. Process according to claim 2 or 3, characterized in that at the start a compound with the formula III is used in which Ar represents a phenyl radical.

6. Process according to any one of the claims 2 to 5, characterized in that at the start a compound with the formula III is used in which one or more of the radicals, $R_2$, $R_3$ and $R_4$ represent a linear, branched or cyclic alkyl radical containing from 1 to 4 carbon atoms.

7. Process according to any one of the claims 2 to 5 characterized in that at the start a compound with the formula III is used in which one or more of the radicals $R_2$, $R_3$ and $R_4$ represent a methyl radical.

8. Process according to any one of the claims 2 to 5, characterized in that at the start a compound with the formula III is used in which $R_3$ and $R_4$ represent a hydrogen atom.

9. Process according to any one of the claims 2 to 8, characterized in that at the start a compound with the formula II is used in which Y represents a hydrogen atom.

10. Process according to any one of the claims 2 to 8, characterized in that at the start a compound with the formula II is used in which Y represents a fluorine atom.

11. Process according to any one of the claims 2 to 10, characterized in that at the start a compound with the formula II is used in which Z represents an oxygen atom.

12. Process according to any one of the claims 2 to 9 and 11, characterized in that at the start an acid is used with the formula $II_1$ :

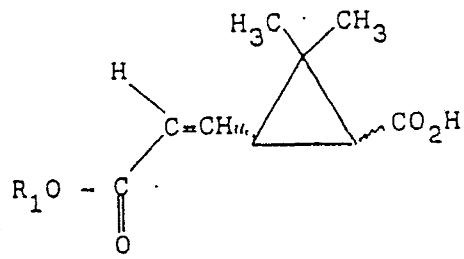

($II_1$)

of 1R, cis structure, the geometry of the double bond being Z, and $R_1$ being defined as previously, or a functional derivative of this acid and a compound with the formula $III_1$ :

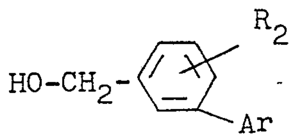

($III_1$)

in which $R_2$ and Ar are defined as previously.

13. Pesticide compositions intended to combat parasites of vegetation, parasites of premises and parasites of warm-blooded animals, characterized in that they contain as active principle at least one compound defined in any one of claims 1 to 12.

14. Insecticide compositions containing as active principle, at least one of the compounds defined in any one of the claims 1 to 12.

15. Acaricide compositions intended to combat parasitic acarids of vegetation, containing as active principle at least one of the compounds defined in any one of the claims 1 to 12.

16. Nematocide compositions containing as active principle, at least one of the compounds defined in any one of the claims 1 to 12.

17. Acaricide compositions intended to combat parasitic acarids of animals, containing as active principle at least one of the compounds defined in any one of the claims 1 to 12.

18. Compositions intended for animal food-stuffs, characterized in that they contain as active principle at least one of the compounds defined in any one of the claims 1 to 12.

19. Combinations endowed with insecticide, acaricide or nematocide activity, characterized in that they contain as active material, on the one hand, one at least of the compounds with the general formula (I), and on the other hand, one at least of the pyrethrinoid esters chosen from the group constituted by the esters of allethrolones, of 3,4,5,6-tetrahydrophthalimidomethyl alcohol, of 5-benzyl-3-furyl methyl alcohol, of 3-phenoxybenzyl alcohol and of α-cyano-3-phenoxybenzyl alcohols with chrysanthemic acids, by the esters of 5-benzyl-3-furyl methyl alcohol with 2,2-dimethyl-3-(2-oxo-3-tetrahydrothiophenylidenemethyl) cyclopropane-1-carboxylic acids, by the esters of 3-phenoxybenzyl alcohol and of α-cyano-3-phenoxybenzyl alcohols with 2,2-dimethyl-3-(2,2-dichlorovinyl) cyclopropane-1-carboxylic acids, by the esters of α-cyano-3-phenoxybenzyl alcohols with 2,2-dimethyl-3-(2,2-dibromovinyl) cyclopropane-1-carboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furyl methyl alcohol, of 3-phenoxybenzyl alcohol and of α-cyano-3-phenoxybenzyl alcohols with 2,2-dimethyl-3-(1,2,2,2-tetrahaloethyl) cyclopropane-1-carboxylic acids, in which « halo » represents a fluorine, chlorine or bromine atom, it being understood that the compounds I can exist in all their possible stereoisomeric forms, as can also the acid an alcohol copulas of the above pyrethrinoid esters.

20. Compositions as defined in any one of the claims 13 to 19, characterized in that they contain as active principle, at least one of the products with the formula I, as defined in claim 2.

**Patentansprüche** (für die Vertragsstaaten : BE, CH, DE, FR, GB, IT, LI, LU, NL, SE)

1. In sämtlichen ihrer möglichen isomeren Formen oder in Form von Gemischen der Isomeren die Verbindungen der Formel (I)

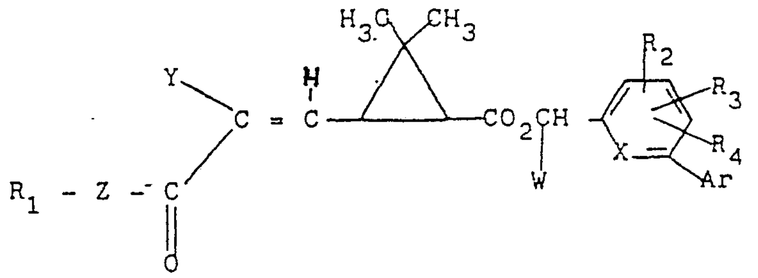

(I)

worin Y ein Wasserstoff- oder Halogenatom oder einen Rest —Oalc oder

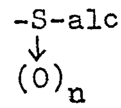

bedeutet, worin alc einen Alkylrest mit 1 bis 6 Kohlenstoffatomen darstellt und n für 0, 1 oder 2 steht, Z ein Sauerstoff- oder Schwefelatom bedeutet, $R_1$

entweder einen gesättigten oder ungesättigten, linearen, verzweigten oder cyclischen Alkylrest mit 1 bis 8 Kohlenstoffatomen, der gegebenenfalls durch eine oder mehrere gleiche oder verschiedene funktionelle Gruppen substituiert ist,

oder eine Arylgruppe mit 6 bis 14 Kohlenstoffatomen, die gegebenenfalls durch eine oder mehrere gleiche oder verschiedene funktionelle Gruppen substituiert ist,

oder einen heterocyclischen Rest bedeutet, der gegebenenfalls durch eine oder mehrere gleiche oder verschiedene funktionelle Gruppen substituiert ist, $R_2$, $R_3$ und $R_4$, die gleich oder verschieden sind, ein Wasserstoffatom, ein Halogenatom, einen Alkylrest mit bis zu 8 Kohlenstoffatomen, einen Haloalkylrest mit bis zu 8 Kohlenstoffatomen, der mehrere Halogenatome enthalten kann, einen Alkoxyrest mit bis zu 8 Kohlenstoffatomen bedeuten, W ein Wasserstoffatom oder einen Cyanorest bedeutet, X ein Kohlenstoffatom oder ein Stickstoffatom bedeutet, Ar einen Phenyl-, Pyridyl-, Thienyl-, Naphthyl-, Furyl- oder Pyrrolylrest bedeutet, der gegebenenfalls substituiert ist, und die ethylenische Doppelbindung die E- oder Z-Geometrie besitzt.

2. Verbindungen der Formel I gemäß Anspruch 1, worin Y ein Wasserstoff- oder Halogenatom bedeutet, $R_2$, $R_3$ und $R_4$ gleich sind, W ein Wasserstoffatom bedeutet und X ein Kohlenstoffatom darstellt.

3. Verbindungen der Formel I gemäß Anspruch 1 oder 2, worin die Struktur der Cyclopropan-Verknüpfung die 1R, cis-Struktur ist.

4. Verbindungen der Formel I gemäß Anspruch 1, 2 oder 3, worin Ar einen Pyrrolylrest bedeutet.

5. Verbindungen der Formel I gemäß Anspruch 1, 2 oder 3, worin Ar einen Phenylrest bedeutet.

6. Verbindungen der Formel I gemäß einem der Ansprüche 1 bis 5, worin einer oder mehrere der Reste $R_2$, $R_3$ und $R_4$ einen linearen, verzweigten oder cyclischen Alkylrest mit 1 bis 4 Kohlenstoffatomen bedeuten.

7. Verbindungen der Formel I gemäß Anspruch 6, worin einer oder mehrere der Reste $R_2$, $R_3$ und $R_4$ einen Methylrest bedeuten.

8. Verbindungen der Formel I gemäß einem der Ansprüche 1 bis 7, worin $R_3$ und $R_4$ ein Wasserstoffatom bedeuten.

9. Verbindungen der Formel I gemäß einem der Ansprüche 1 bis 8, worin Y ein Wasserstoffatom bedeutet.

10. Verbindungen der Formel I gemäß einem der Ansprüche 1 bis 8, worin Y ein Fluoratom bedeutet.

11. Verbindungen der Formel I gemäß einem der Ansprüche 1 bis 10, worin Z ein Sauerstoffatom bedeutet.

12. Verbindungen der Formel I gemäß einem der Ansprüche 1 bis 9 und 11 der Formel

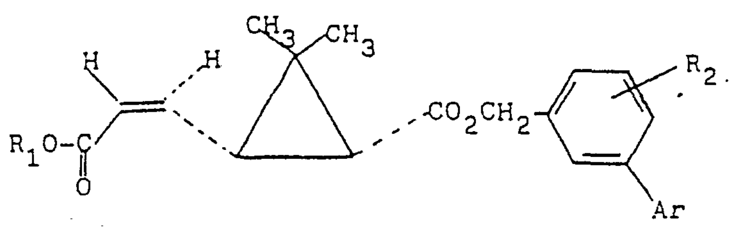

worin die Cyclopropan-Verknüpfung die 1R, cis-Struktur besitzt, die Geometrie der Doppelbindung die Z-Geometrie ist und $R_1$, $R_2$ und Ar die in Anspruch 1 angegebene Bedeutung besitzen.

13. Verfahren zur Herstellung der Verbindungen der Formel I gemäß einem der Ansprüche 1 bis 12, dadurch gekennzeichnet, daß man eine Säure der Formel

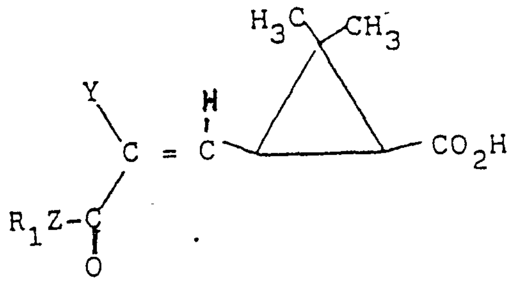

(II)

worin Y, $R_1$ und Z wie in Anspruch 1 definiert sind, oder ein funktionelles Derivat dieser Säure der Einwirkung eines Alkohols der Formel III

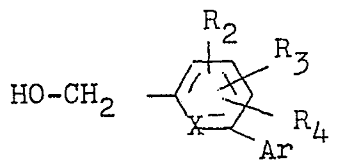

(III)

unterzieht, worin $R_2$, $R_3$, $R_4$, X und Ar wie in Anspruch 1 definiert sind, um die entsprechende Verbindung der Formel I zu erhalten.

14. Verbindungen der Formel I gemäß einem der Ansprüche 1 bis 12 zu deren Verwendung bei der Bekämpfung von Parasiten der Pflanzen, Parasiten von Räumlichkeiten und Parasiten warmblütiger Tiere.

15. Pestizide Zusammensetzungen für die Bekämpfung von Parasiten der Pflanzen, parasiten von Räumlichkeiten und Parasiten warmblütiger Tiere, dadurch gekennzeichnet, daß sie als Wirkstoff zumindest eine Verbindung gemäß einem der Ansprüche 1 bis 12 enthalten.

16. Insektizide Zusammensetzungen, enthaltend als Wirkstoff zumindest eine der Verbindungen gemäß einem der Ansprüche 1 bis 12.

17. Akarizide Zusammensetzungen für die Bekämpfung von parasitären Milben der Pflanzen, enthaltend als Wirkstoff zumindest eine der Verbindungen gemäß einem der Ansprüche 1 bis 12.

18. Nematizide Zusammensetzungen, enthaltend als Wirkstoff zumindest eine der Verbindungen gemäß einem der Ansprüche 1 bis 12.

19. Akarizide Zusammensetzungen zur Bekämpfung von parasitären Milben der Tiere, enthaltend als Wirkstoff zumindest eine der Verbindungen gemäß einem der Ansprüche 1 bis 12.

20. Zusammensetzungen für die tierische Ernährung, dadurch gekennzeichnet, daß sie als Wirkstoff zumindest eine der Verbindungen gemäß einem der Ansprüche 1 bis 12 enthalten.

21. Assoziationen mit insektizider, akarizider oder nemtizider Aktivität, dadurch gekennzeichnet, daß sie als Wirkstoff einesteils zumindest eine der Verbindungen der allgemeinen Formel I und anderenteils zumindest einen der Pyrethrinoidester enthalten, ausgewählt unter den Estern der Allethrolone, des 3,4,5,6-Tetrahydrophthalimidomethylalkohols, des 5-Benzyl-3-furyl-methylalkohols, des 3-Phenoxybenzylalkohols und der α-Cyano-3-phenoxybenzylalkohole der Chrysanthemumsäuren, unter den Estern der 5-Benzyl-3-furylmethylalkohole der 2,2-Dimethyl-3-(2-oxo-3-tetrahydrothiophenylidenmethyl)-cyclopropan-1-carbonsäuren, unter den Estern der 3-Phenoxybenzylalkohole und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern der 3-Phenoxybenzylalkohole der 2-p-Chlorphenyl-2-isopropylessigsäuren, unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, worin « halo » ein Fluor-, Chlor- oder Bromatom bedeutet, wobei die Verbindungen der Formel I in sämtlichen ihrer möglichen stereoisomeren Formen vorliegen können, ebenso wie die Säure- und Alkohol-Verknüpfungen der vorstehenden Pyrethrinoidester.

22. Als neue industrielle Produkte die Produkte der Formel III mit den folgenden Bezeichnungen :
2-(Pyrrol-1-yl)-6-pyridylmethanol ;
(R,S)-Cyano-[2-methyl-3-(pyrrol-1-yl)-phenyl]-methanol.

**Patentansprüche** (für den Vertragsstaat AT)

1. Verfahren zur Herstellung in sämtlichen ihrer möglichen isomeren Formen oder in Form von Gemischen der Isomeren der Verbindungen der Formel (I)

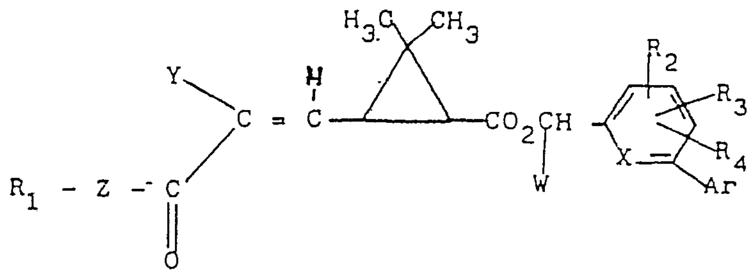

(I)

worin Y ein Wasserstoff- oder Halogenatom oder einen Rest —Oalc oder

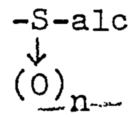

bedeutet, worin alc einen Alkylrest mit 1 bis 6 Kohlenstoffatomen darstellt und n für 0, 1 oder 2 steht, Z ein Sauerstoff- oder Schwefelatom bedeutet, $R_1$

entweder einen gesättigten oder ungesättigten, linearen, verzweigten oder cyclischen Alkylrest mit 1 bis 8 Kohlenstoffatomen, der gegebenenfalls durch eine oder mehrere gleiche oder verschiedene funktionelle Gruppen substituiert ist,

oder eine Arylgruppe mit 6 bis 14 Kohlenstoffatomen, die gegebenenfalls durch eine oder mehrere gleiche oder verschiedene funktionelle Gruppen substituiert ist,

oder einen heterocyclischen Rest bedeutet, der gegebenenfalls durch eine oder mehrere gleiche oder verschiedene funktionelle Gruppen substituiert ist, $R_2$, $R_3$ und $R_4$, die gleich oder verschieden sind, ein Wasserstoffatom, ein Halogenatom, einen Alkylrest mit bis zu 8 Kohlenstoffatomen, einen Haloalkylrest mit bis zu 8 Kohlenstoffatomen, der mehrere Halogenatome enthalten kann, einen Alkoxyrest mit bis zu 8 Kohlenstoffatomen bedeuten, W ein Wasserstoffatom oder einen Cyanorest bedeutet, X ein Kohlenstoffatom oder ein Stickstoffatom bedeutet, Ar einen Phenyl-, Pyridyl-, Thienyl-, Naphthyl-, Furyl-, oder Pyrrolylrest bedeutet, der gegebenenfalls substituiert ist, und die ethylenische Doppelbindung die E- oder Z-Geometrie besitzt, dadurch gekennzeichnet, daß man eine Säure der Formel

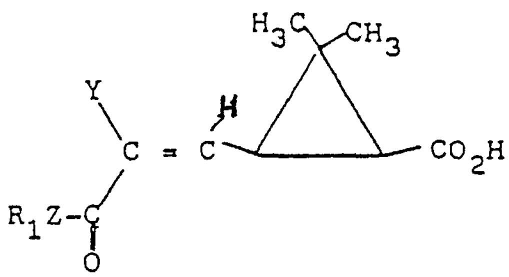

(II)

worin Y, $R_1$ und Z wie vorstehend definiert sind, oder ein funktionelles Derivat dieser Säure der Einwirkung eines Alkohols der Formel III

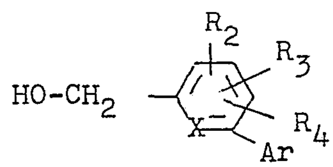

(III)

worin $R_2$, $R_3$, $R_4$, X und Ar wie vorstehend definiert sind, unterzieht, um die entsprechende Verbindung der Formel I zu erhalten.

2. Verfahren gemäß Anspruch 1, dadurch gekennzeichnet, daß man von einer Verbindung der Formel II, worin Y ein Wasserstoff- oder Halogenatom bedeutet, und einer Verbindung der Formel III ausgeht, worin $R_2$, $R_3$ und $R_4$ identisch sind, W ein Wasserstoffatom bedeutet und X ein Kohlenstoffatom darstellt.

3. Verfahren gemäß Anspruch 2, dadurch gekennzeichnet, daß man von einer Verbindung der Formel II mit 1R, cis-Struktur ausgeht.

4. Verfahren gemäß 2 oder 3, dadurch gekennzeichnet, daß man von einer Verbindung der Formel III ausgeht, worin Ar einen Pyrrolylrest bedeutet.

5. Verfahren gemäß Anspruch 2 oder 3, dadurch gekennzeichnet, daß man von einer Verbindung der Formel III ausgeht, worin Ar einen Phenylrest bedeutet.

6. Verfahren gemäß einem der Ansprüche 2 bis 5, dadurch gekennzeichnet, daß man von einer Verbindung der Formel III ausgeht, worin einer oder mehrere der Reste $R_2$, $R_3$ und $R_4$ einen linearen, verzweigten oder cyclischen Alkylrest mit 1 bis 4 Kohlenstoffatomen bedeutet.

7. Verfahren gemäß einem der Ansprüche 2 bis 5, dadurch gekennzeichnet, daß man von einer Verbindung der Formel III ausgeht, worin einer oder mehrere der Reste $R_2$, $R_3$ und $R_4$ einen Methylrest bedeutet.

8. Verfahren gemäß einem der Ansprüche 2 bis 5, dadurch gekennzeichnet, daß man von einer Verbindung der Formel III ausgeht, worin $R_3$ und $R_4$ ein Wasserstoffatom bedeuten.

9. Verfahren gemäß einem der Ansprüche 2 bis 8, dadurch gekennzeichnet, daß man von einer Verbindung der Formel II ausgeht, worin Y ein Wasserstoffatom bedeutet.

10. Verfahren gemäß einem der Ansprüche 2 bis 8, dadurch gekennzeichnet, daß man von einer Verbindung der Formel II ausgeht, worin Y ein Fluoratom bedeutet.

11. Verfahren gemäß einem der Ansprüche 2 bis 10, dadurch gekennzeichnet, daß man von einer Verbindung der Formel II ausgeht, worin Z ein Sauerstoffatom bedeutet.

12. Verfahren gemäß einem der Ansprüche 2 bis 9 und 11, dadurch gekennzeichnet, daß man von einer Säure der Formel $II_1$

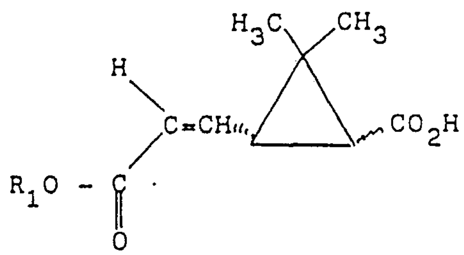

($II_1$)

mit 1R, cis-Struktur, wobei die Geometrie der Doppelbindung die Z-Geometrie ist und worin $R_1$ wie vorstehend definiert ist, oder einem funktionellen Derivat dieser Säure und einer Verbindung der Formel $III_1$

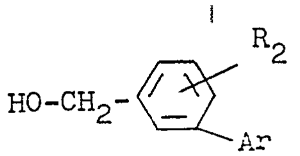

($III_1$)

ausgeht, worin $R_2$ und Ar wie vortehend definiert sind.

13. Pestizide Zusammensetzungen für die Bekämpfung von Parasiten der Pflanzen, Parasiten von Räumichkeiten und Parasiten warmblütiger Tiere, dadurch gekennzeichnet, daß sie als Wirkstoff zumindest eine Verbindung gemäß einem der Ansprüche 1 bis 12 enthalten.

14. Insektizide Zusammensetzungen, enthaltend als Wirkstoff zumindest eine der Verbindungen gemäß einem der Ansprüche 1 bis 12.

15. Akarizide Zusammensetzungen für die Bekämpfung von parasitären Milben der Pflanzen, enthaltend als Wirkstoff zumindest eine der Verbindungen gemäß einem der Ansprüche 1 bis 12.

16. Nematizide Zusammensetzungen, enthaltend als Wirkstoff zumindest eine der Verbindung gemäß einem der Ansprüche 1 bis 12.

17. Akarizide Zusammensetzungen für die Bekämpfung von parasitären Milben der Tiere, enthaltend als Wirkstoff zumindest eine der Verbindungen gemäß einem der Ansprüche 1 bis 12.

18. Zusammensetzungen für die tierische Ernährung, dadurch gekennzeichnet, daß sie als Wirkstoff zumindest eine der Verbindungen gemäß einem der Ansprüche 1 bis 12 enthalten.

19. Zusammensetzungen mit insektizider, akarizider oder nematizider Aktivität, dadurch gekennzeichnet, daß sie als Wirkstoff einesteils zumindest eine der Verbindungen der algemeinen Formel I und anderenteils zumindest einen der Pyrethrinoidester enthalten, ausgewählt unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der Chrysanthemumsäuren, unter den Estern der 5-Benzyl-3-furylmethylalkohole der 2,2-Dimethyl-3-(2-oxo-3-tetrahydrothiophenyliden-methyl)-cyclopropan-1-carbonsäuren, unter den Estern der 3-Phenoxybenzylalkohole und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern der 3-Phenoxybenzylalkohole der 2-p-Chlorphenyl-2-isopropylessigsäuren, unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, worin « halo » ein Fluor-, Chlor- oder Bromatom bedeutet, wobei die Verbindungen der Formel I in sämtlichen ihrer möglichen stereoisomeren Formen vorliegen können, ebenso wie die Säure- und Alkohol-Verknüpfungen der vorstehenden Pyrethrinoidester.

20. Zusammensetzungen gemäß einem der Ansprüche 13 bis 19, dadurch gekennzeichnet, daß sie als Wirkstoff zumindest eines der Produkte der Formel I gemäß Anspruch 2 enthalten.